# EUROPEAN PATENT APPLICATION

(11) **EP 2 908 009 A2**
(43) Date of publication of application: **19.08.2015**
(21) Application number: 15154622.3
(22) Date of filing: 11.02.2015
(51) Int. Cl.: F03G 7/06

(54) **Energy harvesting system for remote monitoring devices**

(30) Priority: 14.02.2014 US 201414181222
(71) Applicant: Rockwell Automation Asia Pacific Business center Pte. Ltd., Singapore 618494 (SG)
(72) Inventor: Mashetty, Srikanth G., Houston, TX Texas 77077 (US); Kidwai, Owais, Tomball, TX Texas 77375 (US); Siado, Celso, Houston, TX Texas 77084 (US); Suheil, Hassan S., Houston, TX Texas 77024 (US)
(74) Representative: Grünecker Patent- und Rechtsanwälte PartG mbB

(57) **Abstract**

A system may include a thermoelectric device that may convert heat energy into electrical energy. The system may also include a pipeline interface that may be disposed on a pipeline, such that the heat energy dissipated from the pipeline is thermally conducted to the thermoelectric device via the pipeline interface. The system may also include a monitoring system that may include one or more sensors that may measure one or more properties associated with an oilfield component. The monitoring system may receive the electrical energy from the thermoelectric device.

## Description

### BACKGROUND

The present disclosure relates generally to generating energy for wireless sensor networks employed in an oilfield environment. More specifically, the present disclosure relates to employing an energy harvesting system in an oilfield environment.

As hydrocarbons are extracted from hydrocarbon reservoirs in oil and/or gas fiields, the extracted hydrocarbons may be transported to various types of equipment, tanks, and the like via a network of pipelines. For example, the extracted hydrocarbons may be transported, via the network of pipelines, from a well site to various processing stations that may perform various phases of hydrocarbon processing to make the produced hydrocarbons available for use or transport.

Information related to the extracted hydrocarbons or related to the equipment transporting, storing, or processing the extracted hydrocarbons may be gathered at a well site or at various locations along the network of pipelines. This information or data may be used to ensure that the well site or pipelines are operating safely and that the extracted hydrocarbons have certain desired qualities (e.g., flow rate, temperature). The data related to the extracted hydrocarbons may be acquired using monitoring devices that may include sensors that acquire the data and transmitters that transmit the data to computing devices, routers, other monitoring devices, and the like, such that well site personnel and/or off-site personnel may view and analyze the data.

To monitor or sense data, the sensors use an energy source, such as a battery, to receive power to perform their monitoring operations. Certain wireless sensors may use a battery as a power source to transmit data acquired by the sensors to other sensors, computing devices, routers, or the like. However, since the amount of energy stored in a battery is limited, it is now recognized that systems and methods for harvesting energy from a surrounding environment of the sensors are desirable.

### BRIEF DESCRIPTION

In one embodiment, a system may include a thermoelectric device that may convert heat energy into electrical energy. The system may also include a pipeline interface that may be disposed on a pipeline, such that the heat energy dissipated from the pipeline is thermally conducted to the thermoelectric device via the pipeline interface. The system may also include a monitoring system that may include one or more sensors that may measure one or more properties associated with an oilfield component. The monitoring system may receive the electrical energy from the thermoelectric device.

In another embodiment, a circuit may include a filter component that may receive electrical energy from a thermoelectric device. The circuit may also include an energy storage device that may receive the electrical energy via the filter component, such that the energy storage device may output a continuous direct current (DC) voltage. The circuit may also include a sensor that may receive the DC voltage via the energy storage device, such that the DC voltage may provide power to the sensor. The sensor may acquire data associated with one or more properties of an oilfield component and send the data to another monitoring system or a router.

In yet another embodiment, an apparatus may include a thermoelectric device that may convert heat energy into electrical energy. The apparatus may also include a pipeline interface that may be coupled to the thermoelectric device, such that the pipeline interface may be disposed on a pipeline. The heat energy dissipated from the pipeline may then be thermally conducted to the thermoelectric device via the pipeline interface. The apparatus may also include a fastener that may couple the pipeline interface to the pipeline.

### DRAWINGS

These and other features, aspects, and advantages of the present invention will become better understood when the following detailed description is read with reference to the accompanying drawings in which like characters represent like parts throughout the drawings, wherein:
FIG. 1 illustrates a schematic diagram of an example hydrocarbon site that may produce and process hydrocarbons, in accordance with embodiments presented herein;
FIG. 2 illustrates a perspective view of an energy harvesting system that may be employed with a monitoring system disposed in the hydrocarbon site of FIG. 1, in accordance with embodiments presented herein;
FIG. 3 illustrates a perspective view of the energy harvesting system of FIG. 2 coupled to a monitoring system disposed in the hydrocarbon site of FIG. 1, in accordance with embodiments presented herein; and
FIG. 4 illustrates a block diagram of circuitry that may be part of the monitoring system disposed in the hydrocarbon site of FIG. 1, in accordance with embodiments presented herein.

### DETAILED DESCRIPTION

One or more specific embodiments will be described below. In an effort to provide a concise description of these embodiments, not all features of an actual implementation are described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

When introducing elements of various embodiments of the present invention, the articles "a," "an," "the," and "said" are intended to mean that there are one or more of the elements. The terms "comprising," "including," and "having" are intended to be inclusive and mean that there may be additional elements other than the listed elements.

Embodiments of the present disclosure are generally directed towards improved systems and methods for providing power to sensors and/or transmitters of a monitoring system employed in a hydrocarbon production and/or processing site. Generally, a hydrocarbon production and/or processing site may include equipment such as pump jacks, electric pumps, manifold-collection centers, separators, storage tanks, pipelines, and the like. Since hydrocarbon production/processing sites are located in remote areas that may be not be easily accessible and may encompass a large amount of area (e.g., land or sea), power sources may not be readily available throughout the hydrocarbon production/processing sites. Moreover, to monitor the vast hydrocarbon production/processing sites, monitoring systems placed at various positions throughout the hydrocarbon production/processing sites to provide information related to properties of the equipment, the hydrocarbons, and the like for a remote user to view and analyze.

Each monitoring system may include a sensor that may acquire data associated with the hydrocarbon production or with the equipment processing the hydrocarbons. The monitoring system may also include a transmitter that may send the data acquired by the sensor to a router, another monitoring system, a computing device, or the like. Since power sources may not be readily available at the hydrocarbon production/processing sites, batteries may provide power to the sensors and the transmitters of the monitoring system. However, the amount of energy stored in a battery is limited and the voltage stored in the battery will eventually decrease, such that the battery may not provide a sufficient amount of power for the components of the monitoring system. As such, a technician may regularly visit the remote locations in which the monitoring systems may be located to check the status of the batteries or replace the batteries of the monitoring systems.

Regularly checking the status or replacing batteries, however, may increase costs associated with monitoring hydrocarbon production/processing sites and may also be an inefficient use of a technician's time. As such, in one embodiment, an energy-harvesting device may provide power to the monitoring system, as opposed to a battery. That is, an energy-harvesting device, such as a thermoelectric device, may be coupled to a heat energy source located in the hydrocarbon production/processing site. The heat energy source may include, for example, a pipeline that transports extracted hydrocarbons from a well to various pieces of equipment in the hydrocarbon production/processing site. The energy harvesting device may convert the temperature difference across its surface into an electrical energy that may be output to a monitoring system that may collect data related to the production or processing of the extracted hydrocarbons.

In certain embodiments, the energy-harvesting device may be coupled to an arc-shaped thermally conductive interface that may couple to a cylindrical shaped pipeline. As such, the arc-shaped thermally conductive interface may physically contact a curved portion of the pipeline to ensure that the heat energy dissipated from the curved portion of the pipeline may be efficiently transferred to the energy-harvesting device.

Moreover, since the hydrocarbon production/processing site may generally be classified as a hazardous location where concentrations of flammable gases, vapors, or dusts may be present, the energy harvester and the monitoring system may be enclosed in an explosion-proof container to ensure that any electrical charge from the energy harvester and the monitoring system's electrical components may be contained within the explosion-proof container. As such, in one embodiment, the monitoring system may include filter circuitry that may receive the electrical energy from the energy harvesting, filter the electrical energy, and store the filtered electrical energy in a battery or the like. By incorporating the filter circuitry in the monitoring system and by enclosing the monitoring system in an explosion proof enclosure, the energy harvester may safely provide power to the sensor and the transmitter in the monitoring system, which may be disposed in a hazardous environment.

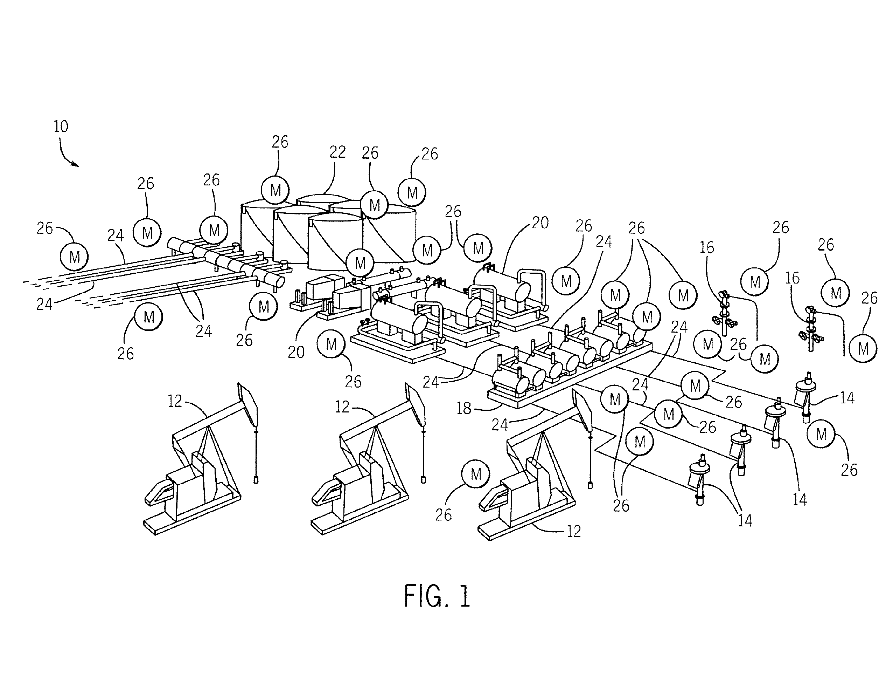

By way of introduction, FIG. 1 illustrates a schematic diagram of an example hydrocarbon site 10. The hydrocarbon site 10 may be an area in which hydrocarbons, such as crude oil and natural gas, may be extracted from the ground, processed, and stored. As such, the hydrocarbon site 10 may include a number of wells and a number of well devices that may control the flow of hydrocarbons being extracted from the wells. In one embodiment, the well devices in the hydrocarbon site 10 may include pumpjacks 12, submersible pumps 14, well trees 16, and the like. After the hydrocarbons are extracted from the surface via the well devices, the extracted hydrocarbons may be distributed to other devices such as wellhead distribution manifolds 18, separators 20, storage tanks 22, and the like. At the hydrocarbon site 10, the pumpjacks 12, submersible pumps 14, well trees 16, wellhead distribution manifolds 18, separators 20, and storage tanks 22 may be connected together via a network of pipelines 24. As such, hydrocarbons extracted from a reservoir may be transported to various locations at the hydrocarbon site 10 via the network of pipelines 24.

The pumpjack 12 may mechanically lift hydrocarbons (e.g., oil) out of a well when a bottom hole pressure of the well is not sufficient to extract the hydrocarbons to the surface. The submersible pump 14 may be an assembly that may be submerged in a hydrocarbon liquid that may be pumped. As such, the submersible pump 14 may include a hermetically sealed motor, such that liquids may not penetrate the seal into the motor. Further, the hermetically sealed motor may push hydrocarbons from underground areas or the reservoir to the surface.

The well trees 16 or Christmas trees may be an assembly of valves, spools, and fittings used for natural flowing wells. As such, the well trees 16 may be used for an oil well, gas well, water injection well, water disposal well, gas injection well, condensate well, and the like. The wellhead distribution manifolds 18 may collect the hydrocarbons that may have been extracted by the pumpjacks 12, the submersible pumps 14, and the well trees 16, such that the collected hydrocarbons may be routed to various hydrocarbon processing or storage areas in the hydrocarbon site 10.

The separator 20 may include a pressure vessel that may separate well fluids produced from oil and gas wells into separate gas and liquid components. For example, the separator 20 may separate hydrocarbons extracted by the pumpjacks 12, the submersible pumps 14, or the well trees 16 into oil components, gas components, and water components. After the hydrocarbons have been separated, each separated component may be stored in a particular storage tank 22. The hydrocarbons stored in the storage tanks 22 may be transported via the pipelines 24 to transport vehicles, refineries, and the like.

The hydrocarbon site 10 may also include monitoring systems 26 that may be placed at various locations in the hydrocarbon site 10 to monitor or provide information related to certain aspects of the hydrocarbon site 10. As such, the monitoring system 26 may be a controller, a remote terminal unit (RTU), or any computing device that may include communication abilities, processing abilities, and the like. The monitoring system 26 may include sensors or may be coupled to various sensors that may monitor various properties associated with a component at the hydrocarbon site 10. The monitoring system 26 may then analyze the various properties associated with the component and may control various operational parameters of the component. For example, the monitoring system 26 may measure a pressure or a differential pressure of a well or a component (e.g., storage tank 22) in the hydrocarbon site 10. The monitoring system 26 may also measure a temperature of contents stored inside a component in the hydrocarbon site 10, an amount of hydrocarbons being processed or extracted by components in the hydrocarbon site 10, and the like. The monitoring system 26 may also measure a level or amount of hydrocarbons stored in a component, such as the storage tank 22. In certain embodiment, the monitoring systems 26 may be iSens-GP Pressure Transmitter, iSens-DP Differential Pressure Transmitter, iSens-MV Multivariable Transmitter, iSens-T2 Temperature Transmitter, iSens-L, Level Transmitter, or Isens-IO Flexible I/O Transmitter manufactured by vMonitor® of Houston, Texas.

In one embodiment, the monitoring system 26 may include a sensor that may measure pressure, temperature, fill level, flow rates, and the like. The monitoring system 26 may also include a transmitter, such as a radio wave transmitter, that may transmit data acquired by the sensor via an antenna or the like. In one embodiment, the sensor in the monitoring system 26 may be wireless sensors that may be capable of receive and sending data signals between monitoring systems 26. To power the sensors and the transmitters, the monitoring system 26 may include a battery or may be coupled to a continuous power supply. Since the monitoring system 26 may be installed in harsh outdoor and/or explosion-hazardous environments, the monitoring system 26 may be enclosed in an explosion-proof container that may meet certain standards established by the National Electrical Manufacturer Association (NEMA) and the like, such as a NEMA 4X container, a NEMA 7X container, and the like.

The monitoring system 26 may transmit data acquired by the sensor or data processed by a processor to other monitoring systems, a router device, a supervisory control and data acquisition (SCADA) device, or the like. As such, the monitoring system 26 may enable users to monitor various properties of various components in the hydrocarbon site without being physically located near the corresponding components.

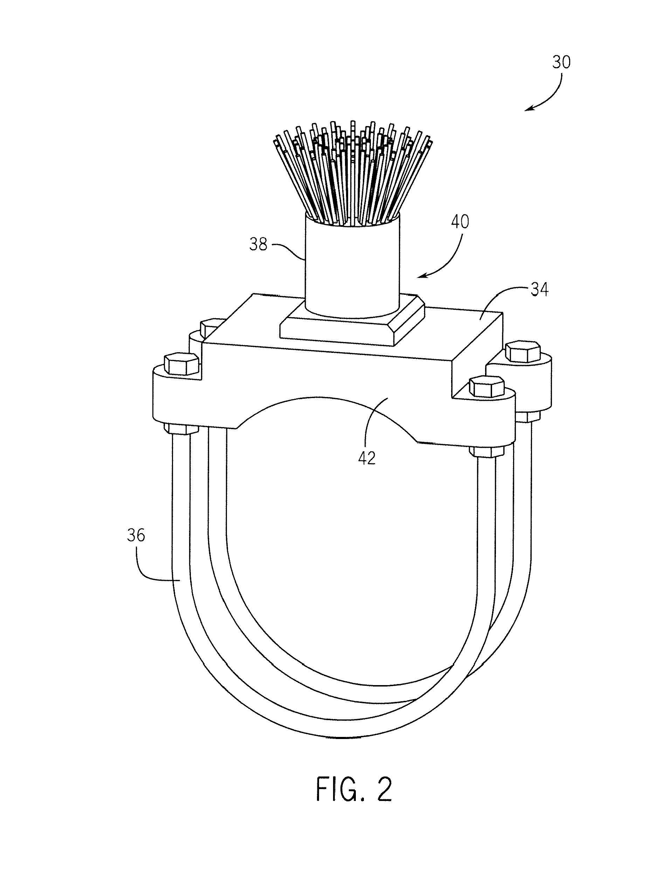

In one embodiment, the monitoring system 26 may be coupled to an energy-harvesting device that may collect energy from its surround environment and generate electrical energy using the collected energy. For instance, FIG. 2 illustrates an example energy harvesting system 30 that may generate electrical energy from heat energy that may be available at various locations within the hydrocarbon site 10. As shown in FIG. 2, the energy harvesting system 30 may include a thermoelectric device 32, a pipeline interface 34, and a fastener 36. As mentioned above, the thermoelectric device 32 may convert temperature differences on two sides of the thermoelectric device 32 into an electrical energy such as a voltage. Generally, an applied temperature gradient across the thermoelectric device 32 may cause charge carriers in the thermoelectric device 32 to diffuse or travel from a side having a higher temperature to a side having a lower temperature. As such, the thermoelectric device 32 may output an unfiltered voltage (i.e., non-continuous, fluctuating) based on the temperature difference between two sides of the thermoelectric device 32. In one embodiment, the unfiltered voltage may be a direct current (DC) voltage that fluctuates based on the energy acquired by the thermoelectric device 32.

As discussed above, the thermoelectric device 32 may be physically coupled to the pipeline 24 disposed in the hydrocarbon site 10. The pipelines 24 generally include an abundant amount of heat energy due to the hydrocarbons transported therein, the temperature of the surround environment, and the like. In one embodiment, the pipeline interface 34 may thermally couple the thermoelectric device 32 to the heat generated by the pipeline 24. As such, the pipeline interface 32 may be composed of a heat conductive material that may efficiently transfer heat energy from one side to another. Example heat conductive materials may include brass, aluminum, or any other metal having relatively high thermal conductivity properties.

As shown in FIG. 2, a first side 38 of the pipeline interface 32 may include a protrusion 39 that may be coupled to a base 40 of the thermoelectric device 32. The top surface of the protrusion 39 may be flat, such that it may be coupled to a flat side of the base 40 of the thermoelectric device 32, thereby maximizing the surface area of the thermoelectric device 32 that may be in contact with the pipeline interface 32. Additionally, since heat is dissipated throughout the pipeline interface 32, the pipeline interface 32 may be designed such that a minimum amount of material is used for portions of the pipeline interface 32 that do not include the protrusion 39. As such, a maximum amount of heat energy received via the pipeline interface 32 may be provided to the thermoelectric device 32.

In one embodiment, the protrusion 39 of the pipeline interface 34 may be designed such that a surface of the base 40 of the thermoelectric device 32 may substantially align with the surface of the protrusion 39 of the pipeline interface 34. That is, the base 40 of the thermoelectric device 32 may be disposed on the surface of the protrusion 39 of the pipeline interface 34, such that the surface area of the base 40 of the thermoelectric device 32 may physically touch a substantial portion of the surface area of the protrusion 39 of the pipeline interface 34.

A second side 42 of the pipeline interface 34 may be shaped as an arc, such that the second side 42 of the pipeline interface 34 may match an arc shape or a portion of the cylindrical shape of the pipeline 24. By having the same arc shape of the pipeline 24, the pipeline interface 34 may cover a large portion of the surface area of a portion of the pipeline 24. The heat energy generated at the surface of this portion of the pipeline 24 may then dissipate through the protrusion 39 of the pipeline interface 34 to the thermoelectric device 32.

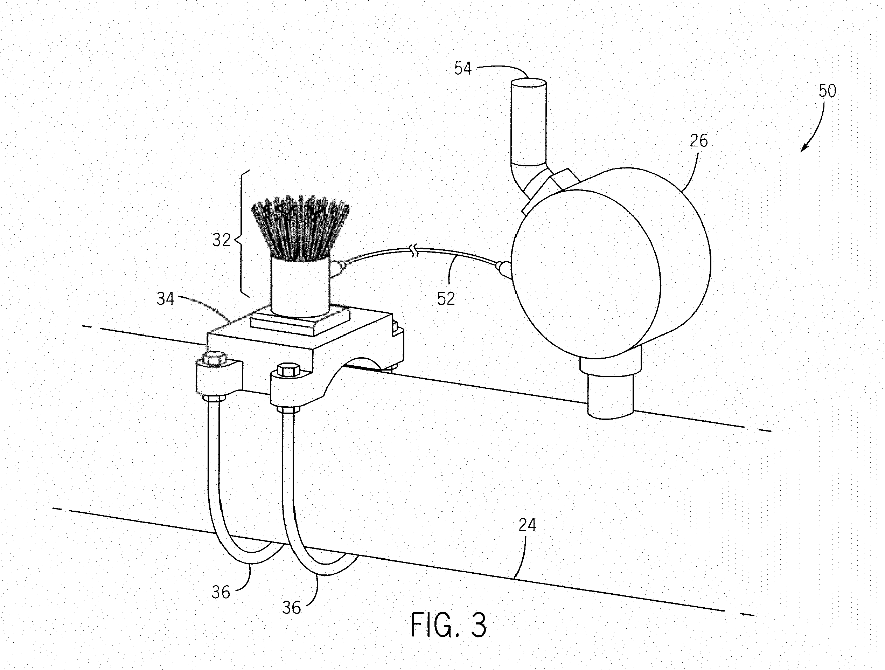

When the pipeline interface 34 is placed on the pipeline 24, the fastener 36 may be coupled around the pipeline 24, as shown in FIG. 3. As such, the fastener 36 may enable the pipeline interface 34 to be fixed onto the pipeline 24. In one embodiment, the pipeline interface 34 may be fixed on top of the pipeline 24 to maximize the amount of heat energy conducted through the pipeline interface 34 since heat energy typically rises. In this manner, the pipeline interface 34 may efficiently thermally conduct the heat energy generated from the pipeline 24 to the thermoelectric device 32. The fastener 36 may be made of any metallic material and may form a loop shape that may match an arc shape or cylindrical shape of the pipeline 24. The fastener 36 may be coupled to the pipeline interface 34 using any type of fastening component, such as a screw, nut, and the like.

FIG. 3 illustrates a perspective view 50 of the energy harvesting system 30 disposed on the pipeline 24 and coupled to the monitoring system 26. In one embodiment, the thermoelectric device 32 may output the electrical energy to the monitoring system 26 via a cable 52. The cable 52 may be composed of any conductive material that may transfer the electrical energy generated by the thermoelectric device 32 to the monitoring system 26.

In one embodiment, the monitoring system 26 may be coupled to the pipeline 24 via hot tapping or pressure tapping, such that sensors of the monitoring system 26 may connect to the pipeline 24 without interrupting the flow of content within the pipeline 24. In any case, the sensors disposed in the monitoring system 26 may include pressure sensors, flow sensors, temperature sensors, and the like. As mentioned above, the sensors in the monitoring system 26 may be wireless sensors that may be capable of receiving and sending data signals between monitoring systems 26, routers, computing devices, and the like. Also mentioned above, the monitoring system 26 may also include a transmitter that may transmit data acquired by the sensor to other monitoring systems 26, routers, computing devices, and the like. The wireless sensors and the transmitter may send and receive data signals via an antenna 54. The antenna 54 may be an electrical device that may convert the data acquired by the sensor into data signals, such as radio waves, that may be transmitted over air.

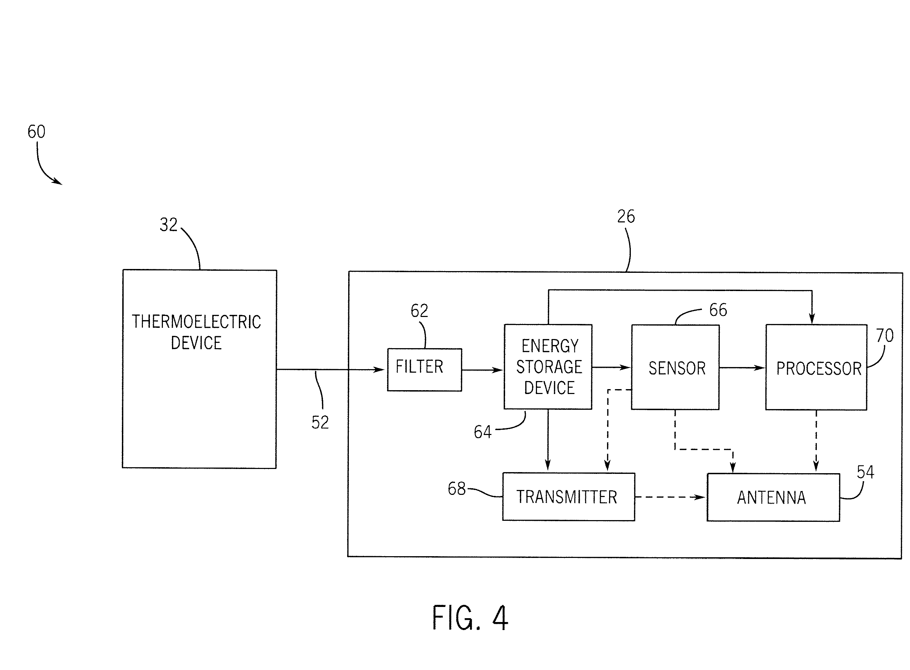

To provide power to the sensors and transmitters disposed in the monitoring system, heat energy from the pipeline 24 may dissipate through the pipeline interface 34 to the thermoelectric device 32. The ambient air or environment may be cooler than the temperature of the pipeline 24. As such, the thermoelectric device 32 may use the temperature difference between the ambient air and the pipeline 24 to generate electrical energy that may be output to the cable 52, which may be coupled to the monitoring system 26. However, as mentioned above, the electrical energy may be an unfiltered voltage signal. Keeping this in mind, FIG. 4 illustrates a block diagram of circuitry 60 that may filter the electrical energy generated by the thermoelectric device 32, such that the electrical energy may be used by components within the monitoring system 24.

Referring now to FIG. 4, the monitoring system 26 may receive the electrical energy generated by the thermoelectric device 32 via the cable 52. As mentioned above, the electrical energy output by the thermoelectric device 32 may be an unfiltered voltage signal. That is, the electrical energy may not be a continuous or reliable source of energy. In one embodiment, the unfiltered electrical energy may be received by a filter component 62 within the monitoring system 26. The filter component 62 may include a low-pass filter that may filters high frequency signals from the received electrical energy. The filter component 62 may then output the filtered signal to charge a storage device, such as a battery.

The filter component 62 may include certain circuitry such as a switch that may couple the electrical energy output by the thermoelectric device 32 to an energy storage device 64. By coupling the electrical energy output by the thermoelectric device 32 to the energy storage device 64, the filter component 62 may enable the electrical energy output by the thermoelectric device 32 to be output to the components of the circuitry 60 as a continuous and reliable form of energy. In one embodiment, the energy storage device 64 may be any type of battery. In another embodiment, the energy storage device 64 may be a super capacitor that may store the energy within the electrical energy output by the thermoelectric device 32.

After the energy is stored in the energy storage device 64, the energy storage device 64 output a steady or continuous DC voltage that may power various electronic components in the monitoring system 26. For example, the energy storage device 64 may be coupled to a sensor 66, which may correspond to the sensor described above that may measure pressure, temperature, fill level, flow rates, and the like. As such, the energy storage device 64 may provide a continuous DC voltage to the sensor 66, such that the sensor 66 may have a sufficient amount of energy to acquire data from the component being monitored and to send the data to a transmitter component 68 or the antenna 54, which may transmit the data acquired by the sensor 66 to other monitoring systems, a router device, a supervisory control and data acquisition (SCADA) device, or the like.

The energy storage device 64 may also be coupled to the transmitter component 68, which may transmit data received from the sensor 66. In one embodiment, the transmitter component 68 may convert the data received from the sensor 66 into data packets, data signals, or the like, such that the transmitter 68 may send the data received from the sensor 66 via the antenna 54 to other monitoring systems, a router device, a supervisory control and data acquisition (SCADA) device, or the like.

The energy storage device 64 may also be coupled to a processor 70. The processor 70 may include any type of processor that may analyze or process data. For example, the processor 70 may process data received from the sensor 66 to determine whether any alarm conditions are present on the component being monitored by the sensor 66 or the like. In one embodiment, the processor 70 may transmit the processed data to other monitoring systems, a router device, a supervisory control and data acquisition (SCADA) device, or the like via the antenna 54

In certain embodiments, the filter component 62 may monitor an amount of charge of energy storage device 64 and may manage the charging of the energy storage device 64. That is, when the energy storage device 64 has a charge that is lower than some low threshold value, the filter component 62 may couple the electrical energy output by the thermoelectric device 32 to the energy storage device 64 until the charge of the energy storage device 64 reaches some upper threshold value. Once the charge of the energy storage device 64 reaches the upper threshold value, the filter component 62 may disconnect the electrical energy output by the thermoelectric device 32 from the energy storage device 64.

By including the filter component 62 and the energy storage device 64 within the circuitry of the monitoring system 26, the filter component 62 and the energy storage device 64 may be enclosed in an explosion-proof container that may be used in the hydrocarbon site 10. That is, the monitoring system 26 may be intrinsically safe to use with the thermoelectric device 32 since the electrical energy output by the thermoelectric device 32 may be contained within the explosion-proof container of the monitoring system 26. As such, the thermoelectric device 32 may be used throughout the hydrocarbon site 10 or other hazardous areas.

While only certain features of the invention have been illustrated and described herein, many modifications and changes will occur to those skilled in the art. It is, therefore, to be understood that the appended claims are intended to cover all such modifications and changes as fall within the true spirit of the invention.

### The following is a list of further preferred embodiments of the invention:

Embodiment 1. A system, comprising:
   a thermoelectric device configured to convert heat energy into electrical energy;
   a pipeline interface configured to be disposed on a pipeline, wherein the heat energy dissipated from the pipeline is thermally conducted to the thermoelectric device via the pipeline interface; and
   a monitoring system comprising one or more sensors configured to measure one or more properties associated with an oilfield component, and wherein the monitoring system is configured to receive the electrical energy from the thermoelectric device.
Embodiment 2. The system of embodiment 1, wherein the pipeline interface comprises a first side having an arc shape.
Embodiment 3. The system of embodiment 2, wherein the arc shape substantially matches a portion of the shape of the pipeline.
Embodiment 4. The system of embodiment 1, wherein the pipeline interface comprises a fastener comprises a loop shape configured to couple the pipeline interface to the pipeline.
Embodiment 5. The system of embodiment 1, wherein the pipeline interface comprises a protrusion, wherein a base of the thermoelectric device is configured to couple to the protrusion.
Embodiment 6. The system of embodiment 1, wherein the pipeline interface comprises a protrusion having a substantially same surface area as a base of the thermoelectric device.
Embodiment 7. The system of embodiment 1, wherein the pipeline interface comprises brass, aluminum, or any combination thereof.
Embodiment 8. The system of embodiment 1, wherein the pipeline interface comprises a thermally conductive metal.
Embodiment 9. The system of embodiment 1, wherein the sensors comprise one or more pressure sensors, one or more temperature sensors, one or more fill level sensors, one or more flow rate sensors, or any combination thereof.
Embodiment 10. The system of embodiment 1, wherein the monitoring system is enclosed in an explosion-proof container.
Embodiment 11. A circuit, comprising:
   a filter component configured to receive electrical energy from a thermoelectric device;
   an energy storage device configured to receive the electrical energy via the filter component, wherein the energy storage device is configured to output a continuous direct current (DC) voltage;
   a sensor configured to:
      receive the DC voltage via the energy storage device, wherein the DC voltage is configured to provide power to the sensor;
      acquire data associated with one or more properties of an oilfield component; and
      send the data to another monitoring system or a router.
Embodiment 12. The circuit of embodiment 11, wherein the filter component is configured couple the electrical energy to the energy storage device when the energy storage device comprises an amount of charge that is lower than a first threshold.
Embodiment 13. The circuit of embodiment 12, wherein the filter component is configured uncouple the electrical energy to the energy storage device when the amount of charge that is greater than a second threshold.
Embodiment 14. The circuit of embodiment 11, wherein the electrical energy comprises an unfiltered DC voltage.
Embodiment 15. The circuit of embodiment 11, wherein the DC voltage is continuous.
Embodiment 16. The circuit of embodiment 11, comprising an explosion-proof container configured to enclose the filter component, the energy storage device, and the sensor.
Embodiment 17. An apparatus, comprising:
   a thermoelectric device configured to convert heat energy into electrical energy;
   a pipeline interface coupled to the thermoelectric device, wherein the pipeline interface is configured to be disposed on a pipeline, and wherein the heat energy dissipated from the pipeline is thermally conducted to the thermoelectric device via the pipeline interface; and
   a fastener configured to couple the pipeline interface to the pipeline.
Embodiment 18. The apparatus of embodiment 17, wherein the pipeline interface comprises a first side having an arc shape that substantially matches a portion of the shape of the pipeline.
Embodiment 19. The apparatus of embodiment 17, wherein the fastener comprises a loop shape configured to match a portion of the shape of the pipeline.
Embodiment 20. The apparatus of embodiment 17, wherein the pipeline interface comprises a protrusion configured to couple to a base of the thermoelectric device.

## Claims

1. A system, comprising:
a thermoelectric device configured to convert heat energy into electrical energy;
a pipeline interface configured to be disposed on a pipeline, wherein the heat energy dissipated from the pipeline is thermally conducted to the thermoelectric device via the pipeline interface; and
a monitoring system comprising one or more sensors configured to measure one or more properties associated with an oilfield component, and wherein the monitoring system is configured to receive the electrical energy from the thermoelectric device.

2. The system of claim 1, wherein the pipeline interface comprises a first side having an arc shape.

3. The system of claim 2, wherein the arc shape substantially matches a portion of the shape of the pipeline.

4. The system of any one of claims 1 to 3, wherein the pipeline interface comprises a fastener comprises a loop shape configured to couple the pipeline interface to the pipeline.

5. The system of any one of claims 1 to 4, wherein the pipeline interface comprises a protrusion, wherein a base of the thermoelectric device is configured to couple to the protrusion.

6. The system of any one of claims 1 to 5, wherein the pipeline interface comprises a protrusion having a substantially same surface area as a base of the thermoelectric device.

7. The system of any one of claims 1 to 6, wherein the pipeline interface comprises brass, aluminum, or any combination thereof.

8. The system of any one of claims 1 to 7, wherein the pipeline interface comprises a thermally conductive metal.

9. The system of any one of claims 1 to 8, wherein the sensors comprise one or more pressure sensors, one or more temperature sensors, one or more fill level sensors, one or more flow rate sensors, or any combination thereof; or
wherein the monitoring system is enclosed in an explosion-proof container.

10. A circuit, comprising:
a filter component configured to receive electrical energy from a thermoelectric device;
an energy storage device configured to receive the electrical energy via the filter component, wherein the energy storage device is configured to output a continuous direct current (DC) voltage;
a sensor configured to:
receive the DC voltage via the energy storage device, wherein the DC voltage is configured to provide power to the sensor;
acquire data associated with one or more properties of an oilfield component; and
send the data to another monitoring system or a router.

11. The circuit of claim 10, wherein the filter component is configured couple the electrical energy to the energy storage device when the energy storage device comprises an amount of charge that is lower than a first threshold.

12. The circuit of claim 11, wherein the filter component is configured uncouple the electrical energy to the energy storage device when the amount of charge that is greater than a second threshold.

13. The circuit of any one of claims 10 to 12, wherein the electrical energy comprises an unfiltered DC voltage; or
wherein the DC voltage is continuous; or
the circuit comprises an explosion-proof container configured to enclose the filter component, the energy storage device, and the sensor.

14. An apparatus, comprising:
a thermoelectric device configured to convert heat energy into electrical energy;
a pipeline interface coupled to the thermoelectric device, wherein the pipeline interface is configured to be disposed on a pipeline, and wherein the heat energy dissipated from the pipeline is thermally conducted to the thermoelectric device via the pipeline interface; and
a fastener configured to couple the pipeline interface to the pipeline.

15. The apparatus of claim 14, wherein the pipeline interface comprises a first side having an arc shape that substantially matches a portion of the shape of the pipeline; or
wherein the fastener comprises a loop shape configured to match a portion of the shape of the pipeline; or
wherein the pipeline interface comprises a protrusion configured to couple to a base of the thermoelectric device.
